Europäisches Patentamt

European Patent Office

Office européen des brevets

(19)

(11) Publication number: **0 324 097 B1**

# EUROPEAN PATENT SPECIFICATION

(12)

(45) Date of publication of patent specification: **23.09.92**   (51) Int. Cl.[5]: **A61F  13/15**

(21) Application number: **88120083.6**

(22) Date of filing: **01.12.88**

(54) **External feminine protection device with skid-resistant coating for holding the device in place.**

(30) Priority: **17.12.87 US 134423**

(43) Date of publication of application:
**19.07.89 Bulletin  89/29**

(45) Publication of the grant of the patent:
**23.09.92 Bulletin  92/39**

(84) Designated Contracting States:
**AT BE CH DE ES FR GB GR IT LI LU NL SE**

(56) References cited:
**EP-A- 0 260 566
DE-C- 616 076
GB-A- 2 187 659
US-A- 3 983 870
US-A- 4 285 343**

(73) Proprietor: **KIMBERLY-CLARK CORPORATION
401 North Lake Street
Neenah, Wisconsin 54956-0349(US)**

(72) Inventor: **Linker III, Paul Means
717 West Prospect Avenue
Appleton, Wisconsin 54911(US)**
Inventor: **Lachapell, Ruth Ann
1077 Mayer Street
Menasha, Wisconsin 54952(US)**

(74) Representative: **Patentanwälte Grünecker,
Kinkeldey, Stockmair & Partner
Maximilianstrasse 58
W-8000 München 22(DE)**

EP 0 324 097 B1

## Description

This invention relates to improvements in the structure of absorbent articles for absorption of human exudate. It particularly relates to pads for feminine hygiene or incontinence.

There has been a variety of devices or appliances configured for catamenial devices. Generally there have been offered two basic kinds of feminine protection device. These are sanitary napkins or pads that have been developed for external use, and tampons that have been developed for residence within the vaginal cavity and interruption of menstrual flow therefrom.

The positioning of feminine hygiene devices so as to stay in the proper location and be comfortable and unobtrusive has been of continuing interest in feminine hygiene. The use of pads or feminine napkins held in place by a belt attached to tabs on the feminine napkins was the traditional method of holdings pads in place for many years. Now, the majority of external feminine products are held in place by utilization of a garment attachment adhesive. Such pressure-sensitive adhesives are placed on the back of the feminine pad and covered by a peel strip that is removed prior to attachment of the pad onto the undergarment of the wearer. The use of such adhesives presents several disadvantages. Among these disadvantages are that the adhesive may stick too firmly to the undergarment and make removal difficult. Another disadvantage is that the adhesive may discolour the undergarment or the pad may tear apart at the time of removal. Further, the cost of adhesives and the peel strips necessary to cover them is a significant portion of the cost of the feminine pad. Adhesives also can cause discomfort if the pad is inadvertently placed upside down in the garment such that the adhesive contacts the body.

Therefore, it would be desirable if a product could be formed that would not require an adhesive, but nevertheless adequately maintain its position within the undergarment of the wearer and in correct placement on the body.

GB-A-2 187 659 discloses a slip-proofing device which comprises a flexible backing web provided with a plurality of projections of a foamed polymer having non-slip properties. The slip-proofing device is formed as a separate unit and has to be attached to the location where its slip-proofing properties are to be used. When used for holding a sanitary napkin in place, the known device has to be sewn or otherwise fixed to the undergarment. This procedure requires separate undergarments to be used when napkins are used, which is more costly for the user and might not be accepted. In addition, this reference does not disclose that the smoothness of the device is of any relevance, and does not disclose any specific value for the coefficient of friction.

US-A-4 285 343 describes a sanitary article containing perforated or roughened areas, in order to provide some frictional engagement with the undergarment. The roughened areas, however, must be used together with common strips of adhesive for attaching the napkin to the inner and the outer side of an undergarment.

Other methods of maintaining pads for absorption of human exudate have been proposed. It has been proposed in US-A-3 881 490 -Whitehead et al. that a pad be provided with a polyurethane foam laminated to the backing element of the pad. An incontinence device is proposed in US-A-4 490 148 - Beckstrom that has a friction-increasing strip fixed to the underside of the garment. In US-A-4 389 211 - Lenaghan the use of a velcro material in contact with the foam outer surface of a feminine pad is utilized as a placement mechanism. However, the above materials suffer from the disadvantage that the foam materials are relatively high in cost and require adhesive connection to the undergarment. Further, such materials have not been shown to be particularly preferred by the users of the garments to which they are attached.

Therefore, there is a continuing need for an improved system for a non-adhesive system for holding catamenial garments in place that is low in cost and effective.

An object of the invention is to overcome the disadvantages of prior systems of holding pads for human exudate in place.

Another object of the invention is to provide a low cost system for holding garments in place.

An additional object is to provide a system of holding garments in place that is unobtrusive.

A further additional object is to provide a system of holding garments in place that will not stain underwear.

These and other objects of the invention are generally accomplished by providing an article for absorption of human exudate that is provided with a coating material that has a high coefficient of friction. Among the preferred coating materials are the coating materials formed from modified acrylics. The materials have a coefficient of friction of greater than 1 as measured by the modified Davis test and a Sheffield smoothness rating of greater than 200 when utilizing a modified ASTM test D-1894.

The invention also includes an embodiment whose skid-resistant surface is placed on at least a portion of the bodyside of an absorbent pad to hold it in place.

Embodiments of the invention are shown in the drawings, wherein

Figures 1, 2, 3 and 4 are top, side, bottom and

2

end views of a pad in accordance with the invention.

Figures 5, 6, 7 and 8 are top, side, bottom and end views of an alternative pad in accordance with the invention.

Figures 9, 10 and 11 are top, side and bottom views of a pad in accordance with the invention.

Figures 12, 13, 14 and 15 are top, bottom, side and perspective views of a coated pad in accordance with the invention.

Figures 16 and 17 are top and cross-sectional views of another embodiment of the invention.

Figures 18 and 19 are top and bottom views of another embodiment of a pad in accordance with the invention.

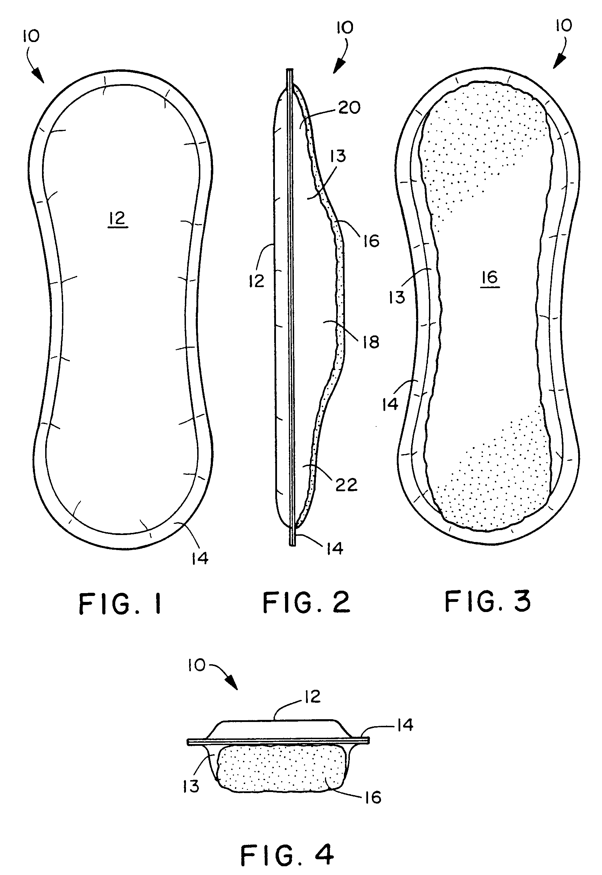

The invention as illustrated by the pad of Figures 1, 2, 3 and 4 is an hourglass-shaped pad 10 that has a generally planar bodyside surface 12 that has the impermeable backing member 13 joined to the cover 12 by adhesive connection in the border area 14. The hourglass-shaped pad has a thicker absorbent in the middle 18 than at the ends 20 and 22. Applied to the lower surface of the pad 10 is a skid-resistant coating 16.

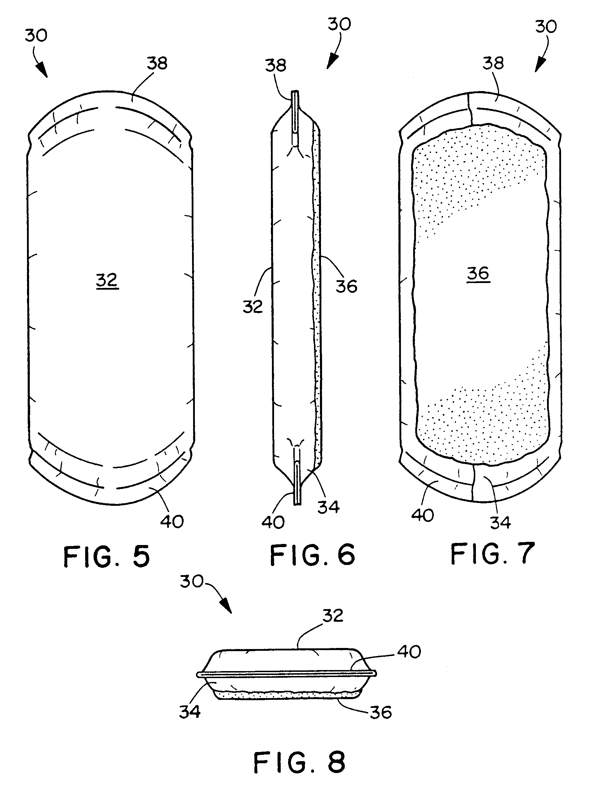

Figures 5, 6 and 7 illustrate a pad 30 that has a permeable bodyside member 32 that is wrapped around the pad, overlapped at the bottom 34 and the overlap sealed with adhesive (not shown). The lower portion of the pad is provided with a skid-resistant coating 36. The pad is sealed at the ends 38 and 40 by heat-fusing of the cover material.

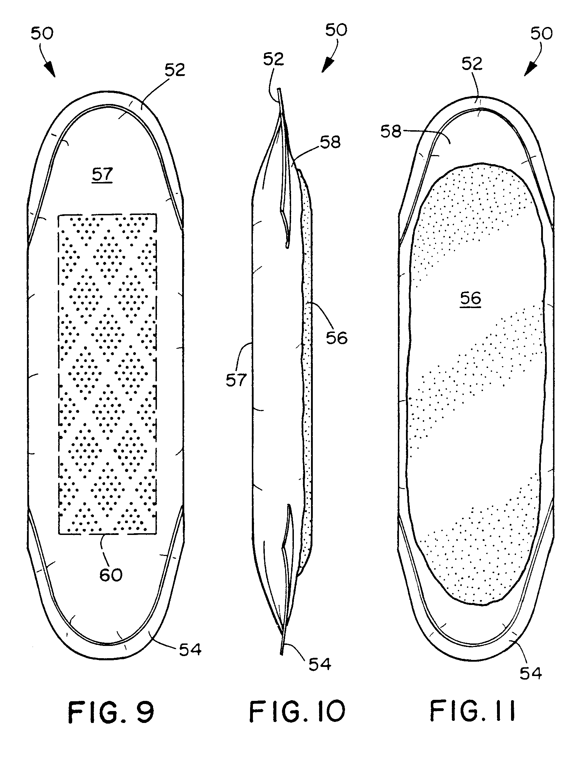

Figures 9, 10 and 11 illustrate a pad 50 having rounded ends 52 and 54. The pad is provided with a skid-resistant coating 56. The bodyside 57 of the pad 50 is provided with a perforated area 60 that aids in flow of material through the permeable cover on the bodyside 57 to the absorbent (not shown) in the interior of the pad 50. The coating 56 covers substantially the entire surface of the back 58 of the pad 50. As used herein top refers to the bodyside of the pad while back refers to the garment side.

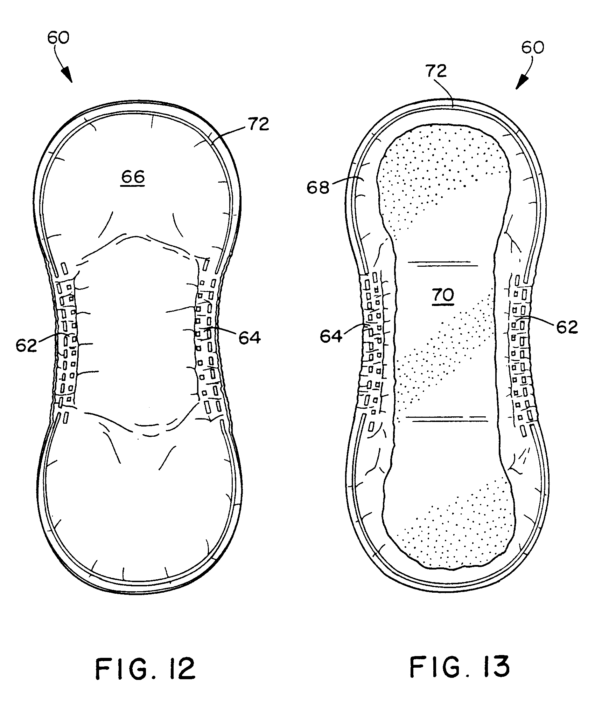

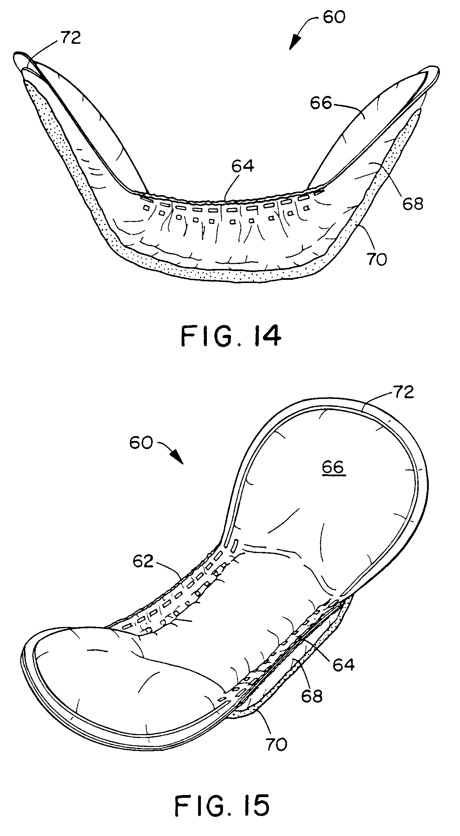

Figures 12, 13, 14 and 15 illustrate a shaped pad 60 in accordance with the invention. The shaped pad is provided with gathered areas 62 and 64 at the sides of the pad that are gathered such that the pad 60 curves to better conform to the body. Areas 62 and 64 further raise to form walls to aid in leakage prevention. The pad is provided with a bodyside permeable member 66 and an impermeable backing member 68. In area 70 a skid-resistant surface coating is provided so that the pad will remain in proper location against the wearer's body. The bodyside permeable member on the top 66 is sealed to the impermeable backing member 68 at heat seal line 72.

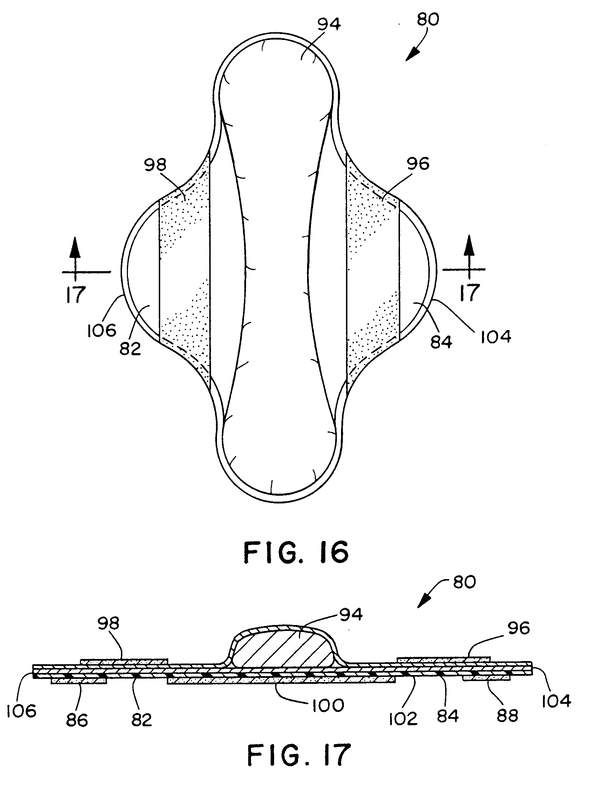

Illustrated in Figures 16 and 17 is a pad 80 having wings 82 and 84 that are intended to be sealed by adhesive areas 86 and 88 either to each other or to the wearer's undergarment. The pad as best illustrated in cross-sectional view of Figure 17 is provided with skid-resistant areas 96 and 98 that will face the bodyside of the pad. These areas 96 and 98 will when the pad is worn be located in the groin area of the wearer and prevent movement of the pad by increasing skid resistance against the body. The absorbent member of the pad 94 is illustrated with an hourglass shape. There is also provided a skid-resistant area 100 on the impermeable backing 102 of the pad. The area 100 prevents movement of the pad against the wearer's garment in order to hold the pad in place in combination with skid-resistant areas 96 and 98 and the overlapped wings 82 and 84 that are held in place by the adhesives 86 and 88. It is also possible that a pad such as illustrated in Figures 16 and 17 would not be provided with adhesive areas 86 and 88 such that the extremities 104 and 106 of the wings hang from the edge of the crotch of the undergarment against the user's thighs rather than be fastened under the crotch of the undergarment. An advantage of the invention is that the pad is not rigidly attached to the undergarment but may move somewhat with the movements of the wearer or with movement of the pad's cover material.

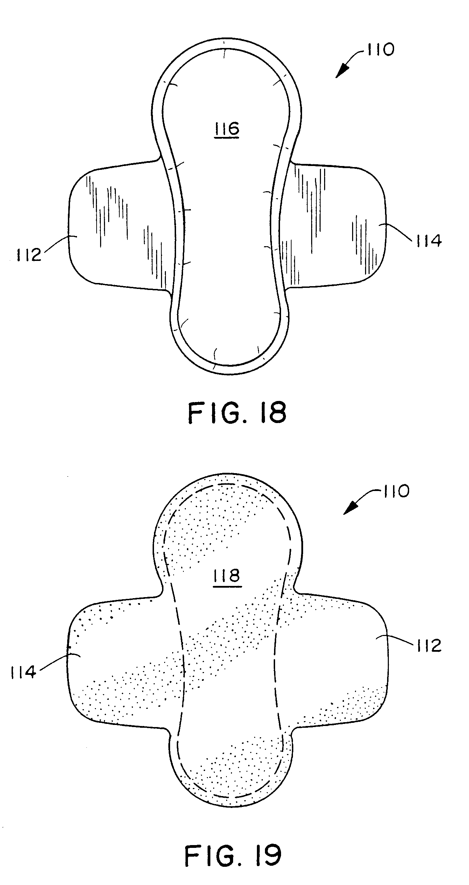

The pad 110 illustrated in Figures 18 and 19 in the top view of Figure 18 and the bottom view of Figure 19 is also provided with wings. The wings 112 and 114 extend outward from the absorbent area 116 on the bodyside and from the back 118. Substantially the entire back area 118 is provided with the skid-resistant surface as is substantially the entire area of wings 112 and 114. This pad is designed such that the skid-resistant surface on 118 and wings 112 and 114 that bear against the wearer's body and wearer's undergarment will hold the pad in place without use of adhesives. As an alternative it is possible that the wings 112 and 114 could be provided with adhesive on the back surface 118 such that they fasten to each other beneath the wearer's underpants.

As illustrated by the above drawings, the coating of skid-resistant materials of the invention may be applied to any of a variety of catamenial pads and adult and children incontinence garments. Further the skid-resistant surface coating may be applied either to the bodyside of the pad or to the back of the pad away from the body. Generally the coating material is applied to a large portion of the pad's back surface. However, depending on the pad's size and the anti-skid properties of the particular coating utilized it may be necessary to only coat a portion of the back surface of the pad.

The skid-resistant surfaces of the invention may be utilized either as the only garment placement system or in combination with a conventional garment attachment adhesive. For instance a small area of garment attachment adhesive could be utilized in order to hold the pad in place while the undergarment was being raised and lowered, but with the skid-resistant surface serving as the primary positioning means for the pad as it was worn. This would result in lower cost as less garment attachment adhesive would be utilized and further would allow the pad to move somewhat with the body rather than being rigidly attached to the undergarment.

The materials suitable for the invention may be any latex or hot melt coating that has sufficient skid-resistant properties to hold a feminine or incontinence pad in place in an undergarment during use of the pad. The materials present a generally smooth, pore-free and nonporous surface after application to the pad. The coefficient of friction of materials has been measured by a Davis Modified form of ASTM test No. D-1894. The D-1894 test calls for a sled wrapped with sponge rubber to be pulled across the test sample at 12,7 mm/min (0.5 inches per minute). The modified Davis test involves wrapping the sled with test samples and pulling them across a Naugahyde sheet at 12,7 mm/min (0.5 inches per minute). Using this test a coefficient of friction of greater than 1 has been found to be satisfactory. A Sheffield smoothness rating of greater than 200 has been found to be suitable. A preferred coefficient of friction is between 1 and 2.5 combined with a smoothness rating of between about 200 and 325.

The coatings used as anti-skid materials of the invention may be any suitable composition. The skid-resistant materials of the invention generally fall into the following groups of materials that include those having adequate skid resistant properties:

- Ethylene vinyl acetate copolymers - could be applied as a hot melt or as a water based coating. Best candidates have at least 28% vinyl acetate
- Polyvinyl acetate - normally used in water-based emulsions
- Styrene-butadiene - applied in an emulsion or as a hot melt
- Cellulose acetate butyrate - normally hot melt coatings
- Ethyl cellulose - normally blended with a plasticizer and a resin and applied as a hot melt
- Acrylics - normally emulsion systems that are not blended
- Synthetic rubber hot melt - (Kraton®) block copolymers having elastomeric and styrenic blocks, rubber, resin, plasticizer blends
- Other hot melts - polyethylenes (alone or blended), polyamides, etc.

Typical of such compositions are the ethylene-vinyl acetate copolymers, acrylic terpolymers of methacrylic acids, acrylic copolymers, ethylene-vinyl acetate/resin latex emulsions, ethylene-vinyl acetate hot-melt adhesives, synthetic rubber (block copolymers with elastomeric and styrenic components) hot melt adhesives, and polyvinyl acetate/resin emulsions. Such materials are available from H. B. Fuller Company, E. 1. Dupont and Findley Adhesives, among others. Compositions of these types have found use as hot-melt and water-based coatings for barrier coatings for nonwovens and/or papers.

While it has been discussed that the coatings of the invention would be placed over an impermeable barrier, it is also possible that the coatings of the invention be placed over a fabric liquid permeable member that would become liquid-impermeable by the action of the coating. This would have the advantage that a low-cost scrim or nonwoven material could be used as the backing with the hot-melt or latex serving both the purpose of forming an impermeable backing member when sealed to the fabric and forming a skid-resistant coating for pad positioning.

The above description has been intended to be illustrative rather than exhaustive of the possibilities of the invention. For instance, while not illustrated the skid-resistant coatings of the invention could be utilized for the products intended for partial labial disposition such as U.S. 4,673,403 - Lassen et al. The skid-resistant coatings also could cover a portion of the absorbent on the ends and/or edges of the bodyside of the pad. The invention is only intended to be limited by the scope of the claims attached hereto.

**Claims**

1. An article (10,30,50,60,80,110) for absorption of human exudate comprising an absorbent positioned between a bodyside layer (12,32,57,66) and a backing layer (13,34,58,68,102) and a nonadhesive, skid-resistant coating (16,36,56,70,96,98,100,118) applied to at least one of said layers for facilitating positioning of said article relative to the anatomy of a user, said coating (16,36,56,70,96,98,100,118) having a coefficient of friction of greater than 1.0 as measured by the modified Davis test and a Sheffield smoothness of greater than about 200.

2. The article of claim 1, wherein said coating (16,36,56,70,96,98,100,118) is selected from the group consisting of ethylene-vinyl acetate copolymers, polyvinylacetate, isobutylene, styrene-butylene, cellulose acetate butyrate, ethyl cellulose, acrylics, synthetic rubber hot melts or emulsions, or mixtures of said materials.

3. The article of claim 1 or 2, wherein said coating (16,36,56,70,96,98,100,118) has a coefficient of friction of between about 1.0 and 2.5.

4. The article of any one of claims 1 to 3, wherein said coating (16,36,56,70,96,98,100,118) has a Sheffield smoothness rating of betwen about 200 to 325.

5. The article of any one of claims 1 to 4, wherein said skid-resistant coating (16,36,56,70,100,118) is applied to the back (13,34,58,68,102) of the pad opposite the bodyside-face (12,32,57,66) of the pad (10,30,50,60,80,110).

6. The article of any one of claims 1 to 4, wherein said skid-resistant coating (96,98) further is applied to a portion of the body-facing side of said pad (80), and directly contacts the body of the user.

7. The article of any one of claims 1 to 4, wherein said coating (96,98,100) is applied to both of said layers for facilitating positioning said article to the anatomy of the user.

8. The article of any one of claims 1 to 7, having two ends, two longitudinal edges, a body facing permeable surface, a back surface and two wings (104,106,112,114) extending from said longitudinal edges a distance sufficient to extend beyond the edges of the crotch of wearer's undergarment wherein said wings (104,106,112,114) are provided with said skid-resistant coating (96,98,118).

9. The article of claim 8, wherein said coating (96,98) is provided on the bodyside of said wings (104,106).

**Patentansprüche**

1. Artikel (10, 30, 50, 60, 80, 110) zum Absorbieren menschlicher Ausscheidungen, mit einem Absorptionsmaterial, das zwischen einer körperseitigen Schicht (12, 32, 57, 66) und einer rückwärtigen Schicht (13, 34, 58, 68, 102) angeordnet ist und einer auf mindestens einer der Schichten zur Erleichterung der Positionierung des Artikels relativ zur Anatomie des Benutzers aufgebrachte, nicht klebende Anti-Gleitbeschichtung (16, 36, 56, 70, 96, 98, 100, 118), wobei die Beschichtung (16, 36, 56, 70,

96, 98, 100, 118) einen Reibungskoeffizienten größer als 1,0, gemessen durch den modifizierten Davis-Test, und eine Glätte nach Sheffield von größer als etwa 200 aufweist.

2. Artikel nach Anspruch 1, wobei die Beschichtung (16, 36, 56, 70, 96, 98, 100, 118) ausgewählt ist aus der Gruppe, die besteht aus Äthylen-Vinyl-Acetat-Copolymere, Polyvenylacetate, Isobutylen, Styrol-Butylen, Zellulose-Acetat-Butyrat, Äthyl-Zellulose, Acryle, Heißschmelzen oder Emulsionen aus synthetischem Gummi, oder aus Mischungen dieser Materialien.

3. Artikel nach Anspruch 1 oder 2, wobei die Beschichtung (16, 36, 56, 70, 96, 98, 100, 118) einen Reibungskoeffizienten von zwischen etwa 1,0 und 2,5 aufweist.

4. Artikel nach einem der Ansprüche 1 bis 3, wobei die Beschichtung (16, 36, 56, 70, 96, 98, 100, 118) eine Glätte-Klassifizierung nach Sheffield zwischen etwa 200 bis 325 aufweist.

5. Artikel nach einem der Ansprüche 1 bis 4, wobei die Anti-Gleitbeschichtung (16, 36, 56, 70, 100, 118) auf die Rückseite (13, 34, 58, 68, 102) der Einlage gegenüber der körperzugewandten Seite (12, 32, 57, 66) der Einlage (10, 30, 50, 60, 80, 110) aufgebracht ist.

6. Artikel nach einem der Ansprüche 1 bis 4, wobei die Anti-Gleitbeschichtung (96, 98) weiterhin auf einen Bereich der dem Körper zugewandten Seite der Einlage (80) aufgebracht ist und den Körper des Benutzers direkt berührt.

7. Artikel nach einem der Ansprüche 1 bis 4, wobei die Beschichtung (96, 98, 100) auf beide Schichten aufgebracht ist, um die Positionierung dieses Artikels relativ zur Anatomie des Benutzers zu erleichtern.

8. Artikel nach einem der Ansprüche 1 bis 7, der zwei Enden, zwei sich in Längsrichtung erstreckende Kanten, eine dem Körper zugewandte, durchlässige Oberfläche, eine rückwärtige Oberfläche und zwei Flügel (104, 106, 112, 114) aufweist, die sich von den Längskanten um einen Abstand wegerstrecken, er ausreicht, um sich um die Kanten des Schrittbereichs der Unterwäsche des Trägers zu erstrecken, wobei die Flügel (104, 106, 112, 114) mit der Anti-Gleitbeschichtung (96, 98, 118) versehen sind.

9. Artikel nach Anspruch 8, wobei die Beschichtung (96, 98) an der dem Körper zugewandten Seite der Flügel (104, 106) vorgesehen ist.

**Revendications**

1. Article (10, 30, 50, 60, 80, 110) pour l'absorption d'exsudat humain comprenant un absorbant situé entre une partie côté peau (12, 32, 57, 66) et une partie de renfort (13, 34, 58, 68, 102) et une couche non adhésive, antiglissement (16, 36, 56, 70, 96, 98, 100, 118) appliquée sur au moins l'une desdites parties pour faciliter la mise en place dudit article en fonction de l'anatomie de l'utilisatrice, ladite couche (16, 36, 56, 70, 96, 98, 100, 118) présentant un coefficient de friction supérieur à 1,0, mesuré par le test de Davis modifié et une douceur Sheffield supérieure à environ 200.

2. Article selon la revendication 1, dans lequel ladite couche (16, 36, 56, 70, 96, 98, 100, 118) est choisie parmi le groupe consitué des copolymères d'acétate d'éthylène-vinyle, polyvinylacétate, isobutylène, styrène-butylène, butyrate d'acétate de cellulose, éthylcellulose, matières acryliques, fusions ou émulsions chaudes de caoutchouc synthétique, ou mélanges desdits matériaux.

3. Article selon la revendication 1 ou 2, dans lequel ladite couche (16, 36, 56, 70, 96, 98, 100, 118) présente un coefficient de friction compris entre environ 1,0 et 2,5.

4. Article selon l'une quelconque des revendications 1 à 3, dans lequel ladite couche (16, 36, 56, 70, 96, 98, 100, 118) présente une valeur Sheffield de douceur comprise entre environ 200 et 325.

5. Article selon l'une quelconque des revendications 1 à 4, dans lequel ladite couche antiglissement (16, 36, 56, 70, 100, 118) est appliquée au dos (13, 34, 58, 68, 102) de la serviette à l'opposé de la partie côté peau (12, 32, 57, 66) de la serviette (10, 30, 50, 60, 80, 110).

6. Article selon l'une quelconque des revendications 1 à 4, dans lequel ladite couche antiglissement (96, 98) est de plus appliquée sur une portion du côté face à la peau de ladite serviette (80), et est en contact direct avec la peau de l'utilisatrice.

7. Article selon l'une quelconque des revendications 1 à 4, dans lequel ladite couche (96, 98, 100) est appliquée sur les deux desdites parties pour faciliter la mise en place dudit article en fonction de l'anatomie de l'utilisatrice.

8. Article selon l'une quelconque des revendications 1 à 7, présentant deux extrémités, deux bords longitudinaux, une surface perméable face à la peau, une surface de renfort et deux protège-côtés (104, 106, 112, 114) suffisamment grands pour aller desdits bords longitudinaux jusqu'au-delà des bords de l'entre-jambes du sous-vêtement de l'utilisatrice dans lequel lesdits protège-côtés (104, 106, 112, 114) sont munis de ladite couche antiglissement (96, 98, 118).

9. Article selon la revendication 8, dans lequel ladite couche (96, 98) est disposée sur le côté peau desdits protège-côtés (104, 106).

FIG. 1

FIG. 2

FIG. 3

FIG. 4

FIG. 5

FIG. 6

FIG. 7

FIG. 8

**FIG. 9**

**FIG. 10**

**FIG. 11**

FIG. 12

FIG. 13

FIG. 14

FIG. 15

FIG. 16

FIG. 17

FIG. 18

FIG. 19